# EUROPEAN PATENT APPLICATION

(11) **EP 4 834 250 A1**
(43) Date of publication of application: **04.11.2026**
(21) Application number: 24911182.4
(22) Date of filing: 25.12.2024
(51) Int. Cl.: A61B 17/3203

(54) **ENDOSCOPE-BASED MONITORING APPARATUS FOR WATER JET MOVEMENT TRAJECTORY**

(30) Priority: 29.12.2023 CN 202311841041
(71) Applicant: Healinno (Beijing) Medical Technology Co., Ltd., Beijing 100176 (CN)
(72) Inventor: ZHAO, Jing, Beijing 100176 (CN); CHEN, Wenbo, Beijing 100176 (CN); SHI, Yilun, Beijing 100176 (CN)
(74) Representative: Patentanwälte Gierlich & Pischitzis Partnerschaft mbB
(86) International application number: PCT/CN2024/142158
(87) International publication number: WO 2025/140258

(57) **Abstract**

The present application provides an endoscope-based monitoring apparatus for a water jet movement trajectory, comprising a water jet, an endoscope, a water jet driving unit, an endoscope driving unit, and a control unit. The water jet driving unit and the endoscope driving unit are both electrically connected to the control unit. The water jet is capable of moving along a planned movement trajectory and spraying a water jet stream to perform tissue resection or ablation, the movement trajectory of the water jet being defined by water jet movement position trajectory parameters. The control unit determines a following movement trajectory of the endoscope based on the planned movement trajectory of the water jet. The endoscope is arranged parallel to the linear movement direction of the water jet, and is located at a rear portion of the water jet, with a predetermined distance S maintained between an observation lens of the endoscope and a water jet spray portion. In a following movement mode, the endoscope follows the water jet along the following movement trajectory to perform at least linear movement, while monitoring actual movement position trajectory parameters of the water jet, wherein the following movement trajectory is defined by following movement position trajectory parameters, which are determined by the control unit based on the planned movement position trajectory parameters of the water jet. The present application enables effective, accurate, and real-time monitoring of execution status during water jet surgical procedures.

## Description

### Technical Field

The present application relates to a monitoring apparatus for a surgery execution instrument, and in particular to an endoscope-based monitoring apparatus for a water jet movement trajectory during an actual surgical procedure.

### Background Art

In recent years, with an advancement of a medical technology, minimally invasive surgery using an image-guided surgery execution instrument such as a water jet has gradually become popular. For example, in minimally invasive surgery using a water jet, an ablation or resection trajectory may be pre-planned based on medical images, allowing the water jet to perform a surgical action along the planned ablation or resection trajectory so as to complete ablation or resection of a tissue.

However, the medical images for the planning are usually images before the resection of the tissue. Even if the planning is performed based on real-time ultrasound images, the real-time ultrasound images only reflect a state before the resection of the tissue. During a surgical procedure, as a resection action progresses, states of the tissue to be resected and surrounding tissues thereof may have changed, and such changes may cause an actual surgery execution trajectory to differ from the pre-planned surgery execution trajectory, resulting in a deviation. A deviation is sometimes caused by an instrument failure, and may occur, for example, when the water jet becomes loose and a driving component unrecognizes this situation and continues to drive movement of the water jet. The deviation is more likely to be unrelated to the instrument failure or the trajectory planning, but rather related to factors such as a surgical environment or a special constitution of a patient. The deviation is not expected to occur for any reason. Reasons are that, on one hand, an occurrence of the deviation means that the execution trajectory deviates from a planned target trajectory, causing the surgery, which should have been performed along the planned trajectory, to lose precision and controllability, on the other hand, the deviation may lead to unnecessary harm to the patient. Therefore, it is necessary to monitor the actual surgery execution trajectory in real time. On one hand, based on a closed-loop feedback mechanism, the surgery is ensured to be performed along the planned target trajectory, which allows adjustment to be performed in time when the occurrence of the deviation is monitored. More importantly, real-time monitoring of the actual surgery execution trajectory can further ensure safety of the surgery, which is especially important in extra protection of sensitive areas such as verumontanum when prostatic hyperplasia resection surgery is performed since damage may still occur even if the surgery is performed along the planned trajectory. On the other hand, achieving accurate and real-time monitoring of the surgery execution trajectory can maximize precise control of the surgical procedure performed according to the plan and minimize a surgical injury.

However, there are many difficulties in real-time monitoring of the actual surgery execution trajectory of the water jet, and a main reason is that, since the water jet is required to spray a high-pressure water jet stream to form a water column region during the surgical procedure, there are a large number of artifacts and noises in the ultrasound images, which causes the real-time ultrasound images to be used only for rough observation and not for accurate calculation of specific parameters related to the actual surgery execution trajectory of the water jet, so that the real-time monitoring of the actual surgery execution trajectory of the water jet cannot be achieved.

Some researchers have also proposed endoscope-based monitoring methods, but since the water jet is in a state of movement during the surgical procedure, a manual control method or an image tracking control method is usually adopted to enable observation of the water jet through the endoscope. However, manual control of the endoscope is time-consuming and laborious, and implementation of a program of an image tracking algorithm is usually complex, making it difficult to achieve real-time and effective tracking of a surgical area. Further, when combined with monitoring and calculation of trajectory parameters, an overall computational load becomes large and a calculation speed becomes slow, making it even more difficult to achieve real-time calculation of the specific parameters related to the actual surgery execution trajectory of the water jet. In addition, the water jet stream sprayed during the surgical procedure using the water jet easily causes blurring of images observed through the endoscope, resulting in reduced clarity of the water jet when the water jet is observed through the endoscope. Further, when the water jet is used in a surgical scenario of resecting the prostatic hyperplasia, surrounding tissues may be compressed, resulting in collapse, during insertion of the endoscope, thereby obstructing an endoscopic field of view, which also leads to a problem of an incomplete field of view when observation is made through the endoscope.

### Summary of the Invention

In order to solve the above technical problems, the present application provides an endoscope-based monitoring apparatus for a water jet movement trajectory, the apparatus including:
a water jet, an endoscope, a water jet driving unit, an endoscope driving unit, and a control unit, in which the water jet driving unit and the endoscope driving unit are both electrically connected to the control unit,
when driven by the water jet driving unit, the water jet is capable of moving along a planned movement trajectory and spraying a water jet stream to perform tissue resection or ablation, the water jet movement trajectory being defined by water jet movement position trajectory parameters, which include linear movement position trajectory parameters, and
when driven by the endoscope driving unit, the endoscope moves and has a following movement mode in which the endoscope is capable of following the water jet along a following movement trajectory to perform at least linear movement and monitoring actual movement position trajectory parameters of the water jet, the following movement trajectory being defined by following movement position trajectory parameters, which at least include linear following movement position trajectory parameters and are determined by the control unit based on planned movement position trajectory parameters of the water jet.

Further, the water jet movement position trajectory parameters further include rotational movement position trajectory parameters and/or jet length trajectory parameters.

Further, linear movement directions of the endoscope and the water jet are parallel to each other, and the endoscope is located at a rear portion of the water jet, with a predetermined distance S maintained between an observation lens of the endoscope and a water jet spray portion.

Further, the water jet movement trajectory is defined as a combination of arrays containing the water jet movement position trajectory parameters for a plurality of steps, the following movement position trajectory of the endoscope is also defined as a combination of arrays containing the following movement position trajectory parameters for the same number of steps, and the control unit determines, based on the water jet movement position trajectory parameters included in each array for one step, the following movement position trajectory parameters of the endoscope in a step corresponding to the step.

Further, the endoscope acquires in real time endoscopic images of a cutting head of the water jet and a water jet stream in the following movement mode, and the control unit analyzes the acquired endoscopic images and determines the jet length trajectory parameters and/or the rotational movement position trajectory parameters in the actual movement position trajectory parameters of the water jet.

Further, the control unit determines the following movement position trajectory parameters based on a tissue collapse visibility distance and/or an optimal endoscope operation distance.

Further, the control unit performs image segmentation processing on an endoscopic image of water jet movement acquired in real time by the endoscope, and monitors the jet length trajectory parameters.

Further, the control unit monitors, by measuring and calculating a swing angle and/or a swing frequency of a water column, the rotational movement position trajectory parameters of the water jet.

Further, the control unit uses, based on a comparison result between a rotation angle range in a preset trajectory of the water jet and a tissue collapse visibility angle range, different monitoring methods for the rotational movement position trajectory parameters of the water jet.

Further, when the entire water column is observable through the endoscope, the control unit monitors and evaluates, by measuring and calculating a swing angle range of the water column in an endoscopic image, whether actual rotational movement position trajectory parameters of the water jet are consistent with planned rotational movement position trajectory parameters.

Further, when the water jet stream is within an endoscopic field of view, the control unit calculates a swing frequency of the water jet based on water jet stream images from the same endoscopic observation angle at an even-numbered step interval among monitoring images of the endoscope, and when the water jet stream is not within the endoscopic field of view, the control unit calculates the swing frequency of the water jet based on feature point images of the water jet from the same endoscopic observation angle at an even-numbered step interval among observation images of the endoscope.

Further, the control unit evaluates, by comparing an actual swing frequency of water column in a step with a swing frequency determined by rotational movement trajectory parameters in the step, whether actual rotational movement position trajectory parameters of the water jet in the step are consistent with planned rotational movement position trajectory parameters.

Further, the endoscope includes a lens having an adjustable tilt angle, and following movement trajectory parameters of the endoscope include rotational following movement position trajectory parameters.

Further, the endoscope further performs safety monitoring on a sensitive part in the following movement mode.

Further, the control unit determines a safe resection depth based on an image acquired by the endoscope, and determines a risk based on a comparison result between a planned jet length trajectory parameter and the safe resection depth.

Further, the control unit determines, based on the image acquired by the endoscope in the following movement mode, the sensitive part, and determines the safe resection depth for the sensitive part based on a shortest distance between a contour boundary of the sensitive part and a cutting head of the water jet.

Further, the control unit further determines the safe resection depth based on a preset safe removal distance.

Further, the control unit performs the safety monitoring on the sensitive part based on rotational movement position trajectory parameters, determines a non-removal region based on an image acquired by the endoscope, and determines a risk based on a comparison result between planned rotational movement position trajectory parameters and the non-removal region.

Further, the endoscope further has an autonomous movement mode in which movement of the endoscope and movement of the water jet are independent of each other, and in the autonomous movement mode, the endoscope is driven to move and parameters are acquired, and the control unit sets or updates the following movement position trajectory parameters based on the acquired parameters.

Further, the endoscope further includes an image acquisition unit configured to acquire an image and transmit the image to the control unit, and the control unit analyzes acquired image data and adjusts the planned movement trajectory of the water jet and the following movement trajectory of the endoscope based on an analysis result.

According to the above solutions proposed in the present application, the technical problems in the background art can be effectively solved, and effective, accurate and real-time monitoring and calculation are performed on specific parameters related to an actual surgery execution trajectory of the water jet during an actual surgical procedure. The present application is particularly applicable to an automatic water jet system, and when the water jet moves along the planned surgery execution trajectory to execute a tissue ablation or resection operation, the solutions of the present application can monitor in real time the actual movement position trajectory parameters of the water jet, which is beneficial for adjustment of the planned movement position trajectory parameters based on a real-time monitoring result. The present application can also monitor an operation region of the water jet during the actual surgical procedure, especially the region involving the sensitive part, assess and determine, based on a real-time monitoring result, a safety risk of the movement trajectory planned in advance, pre-trigger an alarm mechanism or modify a planning scheme before the ablation or resection using the water jet based on a safety risk assessment result so as to reduce damage, and modify the pre-planned movement position trajectory parameters according to the real-time monitoring result, which fully ensures safety of the actual surgical procedure.

### Descriptions of the Drawings

[FIG. 1] FIG. 1 is a schematic diagram of a structure of a surgery execution instrument according to an embodiment of the present application.
[FIG. 2] FIG. 2 is a schematic cross-sectional view of the surgery execution instrument according to the embodiment of the present application.
[FIG. 3] FIG. 3 schematically illustrates a field of view of an endoscope.
[FIG. 4] FIG. 4 schematically illustrates a sensitive part within a lumen of a patient.
[FIG. 5] FIG. 5 schematically illustrates a method for measuring a resection depth of a water jet stream.
[FIG. 6] FIG. 6 illustrates a tiltable endoscope.
[FIG. 7] FIG. 7 illustrates a surgical flow of a tissue resection tool according to an embodiment of the present application.

### Detailed Description

Various exemplary embodiments of the present application are described in detail below with reference to the accompanying drawings. The description of the exemplary embodiments is merely illustrative and is in no way intended to limit the present application and an application or a use thereof. The present application may be implemented in many different forms, and is not limited to the embodiments described herein. These embodiments are provided to make the present application thorough and complete, and to fully describe the scope of the present application to those skilled in the art. It should be noted that, unless otherwise stated, the relative arrangement of components and steps, numerical expressions and values, and the like set forth in these embodiments should be interpreted as merely exemplary and not as limitations. All terms (including technical or scientific terms) used in the present application have the same meaning as understood by those skilled in the art to which the present application belongs, unless otherwise specifically defined. It should also be understood that terms, such as those defined in general dictionaries, should be interpreted as having meanings that are consistent with the meanings in the context of the relevant art, rather than being interpreted with idealized or highly formalized meanings, unless explicitly defined herein. Components, parameters such as specific models of components, and interrelationships and control circuits between components not described in detail in this section may be considered as techniques, methods, and instruments known to those skilled in the art, but where appropriate, the techniques, methods, and instruments should be considered part of the specification.

The term "include", "comprise", or the like used in the present application means that an element before the term covers an element listed after the term, and does not exclude the possibility of covering other elements as well.

In addition, "front", "distal", or "tip" herein indicates a position of a surgical instrument (water jet or endoscope) closer to a tissue or a surgical area, while "rear" or "proximal" indicates a position of the surgical instrument (water jet or endoscope) closer to an operator.

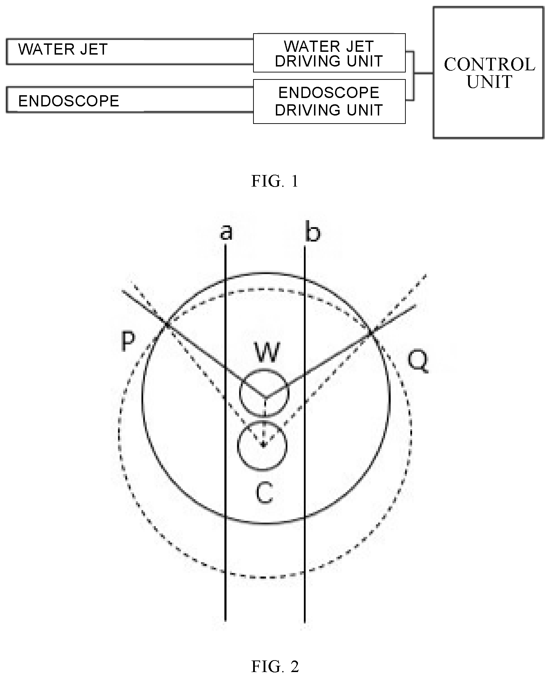

FIG. 1 is a schematic diagram of a structure of a surgery execution instrument according to an embodiment of the present application. Typically, the surgery execution instrument includes a water jet and a monitoring apparatus for a movement trajectory thereof. As shown in FIG. 1, the surgery execution instrument includes a water jet and an endoscope at a front end, and a water jet driving unit, an endoscope driving unit 4, and a control unit at a rear end. The control unit is electrically connected to the water jet driving unit that is drivingly connected to the water jet, and is further electrically connected to the endoscope driving unit that is drivingly connected to the endoscope.

The water jet includes a sheath and a movable high-pressure resistant pipeline disposed in the sheath, and a front end of the high-pressure resistant pipeline is capable of spraying a high-pressure water jet stream to ablate or resect a tissue. In the present application, a cutting head of the water jet refers to a front end (also referred to as a water jet spray portion or nozzle) of the high-pressure resistant pipeline from which the water jet stream is sprayed, and a portion of the high-pressure resistant pipeline other than the cutting head is also referred to as a lance. In some contexts, there is no strict distinction between the water jet and the water jet cutting head. In addition, in the present application, "ablation" and "resection" are not strictly distinguished since the meanings thereof are similar, and in some contexts, the "ablation" and "ablation" are also referred to as "removal"; and "water jet stream" and "water column" are not strictly distinguished, and both refer to the high-pressure water jet stream sprayed from the cutting head of the water jet.

The control unit is electrically connected to the water jet driving unit, and when the water jet is driven by the water jet driving unit, the water jet enters a target tissue region (through an urethra or a perineum in a case of prostatic hyperplasia resection surgery), and executes ablation or resection by spraying the high-pressure water jet stream.

The water jet moves along a planned path, and the planned path may be a path designed in advance by a doctor or a processor based on image information. A trajectory formed by moving along the planned path is a planned movement trajectory, and the planned movement trajectory may be a movement control position trajectory generated by an image planning module performing fitting based on continuous boundary position trajectories pre-planned on a navigation image.

The water jet can perform linear and/or rotational movement when driven by the water jet driving unit, the linear movement of the water jet refers to a process in which the water jet advances or retreats in a linear direction defined by a sheath channel of the water jet, the rotational movement of the water jet refers to rotation of the water jet relative to an axial direction of the lance of the water jet. The cutting head sprays the water jet stream while the water jet rotates, thereby ablating or removing a target tissue in a scanning manner.

While the water jet performs the linear and/or rotational movement, the cutting head of the water jet sprays the high-pressure water jet stream. A length of the water jet stream indicates a distance of an action point of the water jet stream from the cutting head. Since the length of the water jet stream is controllable by the pipeline and a hydraulic power module, the length of the water jet stream is also an important parameter for describing the movement trajectory of the water jet.

In order to represent the above movement process of the water jet, in the present application, the water jet movement trajectory is defined by movement position trajectory parameters of the water jet. The movement position trajectory parameters of the water jet represents a position of the action point of the water jet stream of the water jet in a water jet coordinate system. A movement control module can enable the water jet to move according to preset movement position trajectory parameters through a water jet movement control motor, a pipeline, and a hydraulic pump of the hydraulic power module. The preset movement position trajectory parameters may be determined based on a planned movement trajectory.

Preferably, linear movement position trajectory parameters representing the linear movement of the water jet may be a position of the action point on a jet long axis and/or a velocity of the action point on a jet long axis in a water jet coordinate system, rotational movement position trajectory parameters representing the rotational movement of the water jet may be a cross-sectional angle of the action point and/or a cross-sectional rotational speed, and jet length trajectory parameters representing the length (that is, the distance between the water jet spray portion and the action point) of the water jet stream sprayed by the cutting head of the water jet may be a jet length of the action point.

In the present application, the endoscope is set to have a following movement mode, and therefore provided near the water jet. Axes of the endoscope and the water jet are parallel to each other, and linear movement directions of the endoscope and the water jet are also parallel to each other.

Various fixed installation methods can be adopted to provide the endoscope near the water jet. For example, the endoscope and the water jet can be provided in a manner of sharing a sheath, with the endoscope and the water jet respectively positioned in two parallel channels defined by the sheath. Alternatively, the endoscope and the water jet can each include an own independent sheath, but the sheath of the endoscope and the sheath of the water jet can be integrally formed, or the two can be fixedly installed relative to the same base. The endoscope and the water jet are provided in a manner of sharing the sheath or the sheaths of the endoscope and the water jet are integrally formed, which makes it easier to fix relative movement paths of the endoscope and the water jet, thereby ensuring consistency between a following movement trajectory of the the endoscope and a water jet movement trajectory, and thus facilitating stability of following movement of the endoscope.

The control unit is further electrically connected to the endoscope driving unit, and when driven by the endoscope driving unit, the endoscope also enters the target tissue region. The endoscope captures images of the water jet and a surrounding tissue environment thereof, acquires images within the endoscopic field of view in real time, and transmits acquired endoscopic image data to the control unit.

The endoscope can perform linear and/or rotational movement when driven by the endoscope driving unit, the linear movement thereof is a process in which the endoscope advances or retreats in a linear direction defined by a sheath channel of the endoscope, and the rotational movement thereof is optional, for example, the endoscope is rotated relative to an axial direction thereof in order to adjust the endoscopic field of view.

In the present application, the endoscope has the following movement mode in which the endoscope can follow the water jet along the following movement trajectory to perform at least the linear movement, and the linear movement of the endoscope is synchronized with the linear movement of the water jet.

In a following movement mode in some embodiments, the endoscope is set to follow only the linear movement of the water jet, but not the rotational movement thereof, so as to facilitate observation of both the linear and rotational movement of the water jet from a fixed viewing angle through the endoscope.

In a following movement mode in some other embodiments, the endoscope is set to follow not only the linear movement of the water jet but also the rotational movement thereof, so as to ensure that both the water jet in the rotational movement and the water column are within the endoscopic field of view.

In order to achieve the following movement mode of the endoscope, the control unit generates the following movement trajectory of the endoscope based on the planned movement trajectory of the water jet, and sends the generated following movement trajectory of the endoscope to the endoscope driving unit so as to control the endoscope to move along the following movement trajectory.

The following movement trajectory of the endoscope is defined by following movement position trajectory parameters that at least include linear following movement position trajectory parameters. The linear following movement position trajectory parameters are determined based on the linear movement position trajectory parameters of the water jet.

The endoscope may further have an autonomous movement mode in which the endoscope independently performs the linear and/or rotational movement within the sheath channel of the endoscope, independent of the linear and/or rotational movement of the water jet.

A user may select a movement mode of the endoscope through the control unit or a similar mechanism to meet requirements for different scenarios. For example, during a preoperative instrument debugging phase, the user can select the endoscope to enter the autonomous movement mode, manually control or drive, with a motor, the endoscope to move within the sheath channel thereof, thereby acquiring relevant parameters and setting or updating the following movement position trajectory parameters based on the acquired parameters. When the water jet is in place and ready to start an elimination or ablation operation on the tissue, the endoscope is selected to enter the following movement mode in which the endoscope is driven by the motor to move, which can achieve real-time monitoring of actual movement position trajectory parameters during an operation of the water jet.

Preferably, an image acquisition unit of the endoscope acquires an image and transmits the image to the control unit, the control unit analyzes image data after acquiring the image data, and adjusts the planned movement trajectory of the water jet and the following movement trajectory of the endoscope based on an analysis result of the image data.

The control unit may convert the planned movement trajectory or parameters into the movement position trajectory parameters, for example, convert a movement trajectory or parameters planned by a doctor in an image coordinate system into a movement control position trajectory or parameters, and further convert the movement control position trajectory or parameters into the movement position trajectory parameters in the water jet coordinate system, or directly convert the received planned movement trajectory or parameters or movement control position trajectory or parameters into the movement position trajectory parameters in the water jet coordinate system.

The control unit may directly acquire water jet movement position trajectory parameters which are determined based on a pre-planned movement trajectory, and send, to the water jet driving unit, the water jet movement position trajectory parameters which are determined based on the pre-planned movement trajectory so as to drive the water jet to move according to the water jet movement position trajectory parameters.

The movement position trajectory parameters include the linear movement position trajectory parameters, the rotational movement position trajectory parameters, and the jet length trajectory parameters.

The linear movement position trajectory parameters may be a position z of the action point on the jet long axis and/or a velocity Vz of the action point on the jet long axis in the water jet coordinate system, the rotational movement position trajectory parameters may be a cross-sectional angle θ of the action point and/or a cross-sectional rotational speed V_{θ} in the water jet coordinate system, and the jet length position trajectory parameters may be a jet length R of the action point in the water jet coordinate system.

Further, the linear movement position trajectory parameters may include a linear speed Vz, a linear start position z_start, a linear stop position z_stop, and the like, the rotational movement position trajectory parameters may include a rotational speed V_{θ}, a rotational start angle θ_start, a rotational stop angle θ_stop, and the like, and the jet length trajectory parameters may include a jet length R, a resection depth D, and the like.

An overall movement trajectory of the water jet can be divided into a plurality of steps, and movement position trajectory parameters are assigned for each step. The movement position trajectory parameters of each step may be represented by an array, for example: [{stepNO, z_start, z_stop, Vz, θ_start, θ_stop, V_{θ}, R}], in which stepNO is a step number of water jet movement, z_start is a linear start position of the water jet in the step, z_stop is a linear stop position of the water jet in the step, Vz is a linear speed of the water jet movement in the step, θ_start is a rotational start angle of the water jet in the step, θ_stop is a rotational stop angle of the water jet in the step, V_{θ} is a rotational speed of the water jet in the step, and R is a jet length of the water jet in the step. Alternatively, the resection depth D of the water jet in the step can be used for representing the jet length trajectory parameter in the step. In the present application, the resection depth D and the jet length R are considered to be approximately equal parameters.

By representing the movement position trajectory parameters of the water jet with the array of each step, a combination of arrays, each containing the movement position trajectory parameters for one step, can be used for describing the partial or overall movement trajectory of the water jet.

A length of each step may be appropriately set according to a surgical scenario and a surgical target. For example, in the case of prostatic hyperplasia resection or ablation surgery, the length of each step of the movement trajectory of the water jet is set to be in the order of mm, which can basically meet precision requirements for surgical procedure control, thereby achieving precise and controllable resection control. For scenarios where surgical precision requirements are not high, the length of each step may be set to a larger value.

In order to use the endoscope to monitor the actual movement position trajectory parameters during the operation of the water jet, in the present application, the endoscope is set to have the following movement mode in which the endoscope follows the water jet along the following movement trajectory. Therefore, it is necessary for the control unit to determine the following movement trajectory of the endoscope in advance based on the planned movement trajectory of the water jet. Specifically, the following movement position trajectory parameters of the endoscope are determined based on the planned movement position trajectory parameters of the water jet.

For the case in which the overall movement trajectory of the water jet is divided into the plurality of steps and the movement position trajectory parameters are assigned for each step, the following movement trajectory of the endoscope is also divided into the same number of steps, following movement position trajectory parameters of the endoscope for each step are generated based on the movement position trajectory parameters for each step of the water jet, and a combination of arrays, each containing the following movement position trajectory parameters for one step of the endoscope, is used for describing the following movement trajectory of the endoscope.

Specifically, the movement position trajectory parameters of the water jet in each step are represented by, for example, [{stepNO, z_start, z_stop, Vz, θ_start, θ_stop, V_{θ}, R}], and the following movement position trajectory parameters of the endoscope in each step are represented by [{CstepNO, Cz_start, Cz_stop, CVz}]. Based on the step number stepNO in the planned movement position trajectory parameters of the water jet, a step number CstepNO of a corresponding following movement of the endoscope is determined. Based on the linear start position z_start of the water jet in the step, a linear start position Cz_start of the endoscope in the step is determined. Based on the linear stop position z_stop of the water jet in the step, a linear stop position Cz_stop of the endoscope in the step is determined. Based on the linear movement speed Vz of the water jet, a linear movement speed CVz of the endoscope is determined.

Preferably, the step and the linear movement speed of the endoscope are consistent with those of the water jet, and CstepNO = stepNO and CVz = Vz can be set. Further, a linear movement time of the endoscope within each step is preferably set to be consistent with a linear movement time of the water jet within each step. Therefore, the linear movement speed CVz of the endoscope can be calculated by CVz = Vz × (Cz_stop - Cz_start)/(z_stop - z_start).

Since the water jet spray portion of the water jet continuously sprays the water jet stream in an operation state to form a radial water column (usually a gas-liquid mixture containing cavitation bubbles), if an observation lens of the endoscope is below the water jet spray portion, the field of view is inevitably disturbed by the radial water column, making clear observation impossible, and if the observation lens of the endoscope is provided at the front of the water jet spray portion, this means that a movement region of the endoscope exceeds a movement region of the water jet, so that the movement region of the water jet is limited at the same insertion depth. In order to achieve clear observation of the water jet through the endoscope, in the present application, the endoscope is located at a rear portion of the water jet, with a predetermined distance S maintained between the observation lens of the endoscope and the water jet spray portion. In this way, endoscopic observation can avoid interference caused by the sprayed water jet stream and contribute to providing an unrestricted and improved field of view and observation effect of a surgical area. Further, after S is determined, the linear start position Cz_start of each step in the following movement position trajectory parameters of the endoscope can be determined based on the linear start position z_start of each step in the planned movement position trajectory parameters of the water jet, and the linear stop position Cz_stop of each step in the following movement position trajectory parameters of the endoscope can be determined based on the linear stop position z_stop of each step in the planned movement position trajectory parameters of the water jet. Specifically, during the prostatic hyperplasia resection surgery, the water jet and the endoscope move synchronously to the most distal position (taking the position as a start point), the endoscope is driven to retreat to the rear of the water jet spray portion, specifically at the distance S behind the water jet, and correspondingly, the linear start position Cz_start = z_start + S and the linear stop position Cz_stop = z_stop + S of the endoscope in the following movement trajectory parameters of the endoscope are set.

The endoscope is disposed at a certain distance behind the water jet in an axial direction, so that the water jet can spray the water jet stream without obstruction, and the endoscope does not obstruct the water jet stream sprayed by the water jet, which makes it possible for the endoscope to effectively capture images of the water jet stream of the water jet. However, it is not enough to only provide the endoscope at a predetermined distance behind the water jet, and a determination method of S is an important factor for achieving the clear observation.

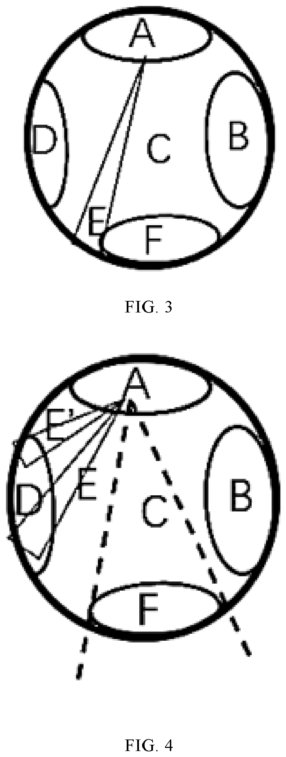

FIG. 3 is a schematic diagram of a visible region within the endoscopic field of view. As shown in FIG. 3, C represents an approximate center of the endoscope, A represents an image of the water jet in the endoscopic field of view, and E represents the water jet stream, that is, the water column, sprayed from the water jet spray portion. When the following movement position trajectory parameters of the endoscope are determined, it is necessary to ensure that the water jet A and the water jet stream E can be clearly observed within the endoscopic field of view, thereby using image information of the water jet A and the water jet stream E for calculation processing.

Since different endoscopes have different imaging capabilities, in order to ensure clear imaging, the present application introduces a parameter, that is, an optimal endoscope operation distance L, when a distance between the endoscope lens and the cutting head of the water jet is determined. The optimal endoscope operation distance L represents a distance from an object to the endoscope lens at which the object can be clearly imaged and which is typically related to a focal length of the endoscope. In the present application, the optimal endoscope operation distance L represents an optimal operation distance between the endoscope lens and the cutting head of the water jet, and within this distance range, the endoscope can clearly image the cutting head A of the water jet and the water column E within the field of view thereof. L can be determined in advance through experiments. For example, in the autonomous movement mode, the water jet stops moving after being driven to enter the target tissue region, and the endoscope is then driven to advance to the vicinity of the cutting head of the water jet within the sheath channel of the endoscope and reciprocates until reaching a region where images of the cutting head A of the water jet and the water column E can be clearly observed through the endoscope, a position of which is recorded, whereby the optimal endoscope operation distance L is determined based on the recorded position.

The optimal endoscope operation distance L may be a numerical range or a set of dynamically changing values related to a position of the water jet, that is, different optimal endoscope operation distances L can be determined for different fixed positions P of the water jet, and an array corresponding to the optimal endoscope operation distance L and the position P of the water jet can be stored.

The following movement position trajectory parameters of the endoscope can be determined based on the optimal endoscope operation distance L, for example, the linear start position Cz_start = z_start + L and the linear stop position Cz_stop = z_stop + L of the endoscope in the linear following movement position trajectory parameters of the endoscope are set.

When the water jet is inserted into a human tissue, with the insertion of the water jet, a tissue around the water jet collapses. Especially in the prostatic hyperplasia resection surgery, the water jet is inserted through the urethra, and surrounding prostatic hyperplasia tissues contract and collapse due to compression and gathers around the water jet, as shown in FIG. 3. B, D, and F represent tissues that are present in the endoscopic field of view due to compression and deformation of the surrounding tissues caused by the insertion of the water jet.

The inventors have found through repeated experiments that, since the compressed tissue is soft and deformable, the resulting obstruction of the endoscopic field of view occurs during a movement of a surgical instrument. A degree of obstruction is related to a volume and texture of the tissue, as well as a distance between the endoscope and the cutting head of the water jet. For example, when the endoscope is close to the cutting head of the water jet, since the cutting head of the water jet inserted in front provides a certain supporting and expanding effect on the surrounding tissues, a tissue collapse effect is almost not present or minimal within the endoscopic field of view, and does not affect the observation. When the distance between the endoscope and the cutting head of the water jet is far, the supporting and expanding effect on the surrounding tissues of cutting head of the water jet inserted in front disappears, and the tissue collapse effect around the lance is more obvious. In severe cases, the endoscopic field of view is almost completely obstructed.

In order to solve the problem, the present application introduces a parameter, that is, a tissue collapse visibility distance T, which is the distance between the endoscope and the cutting head of the water jet and used for representing an impact of the tissue collapse effect on the endoscopic field of view. The tissue collapse visibility distance T at each of different tissue sites in the ultrasound image during the insertion of the water jet is obtained in advance through experiments. For example, in the autonomous movement mode, the water jet stops moving after being driven to enter the target tissue region, the endoscope gradually move away from the cutting head of water jet after being driven to advance within the endoscope sheath channel until the endoscope is directly below the cutting head of the water jet, and the water column E can be observed in the endoscopic field of view within the tissue collapse visibility distance T. Beyond this distance, the endoscopic field of view is obstructed due to an impact of tissue contraction, compression, and encapsulation. A position where the endoscopic field of view goes from being unobstructed to beginning to be obstructed or being obstructed in a small part is critical position, and a distance between the endoscope and the cutting head of water jet which corresponds to the position is recorded as the tissue collapse visibility distance T. In other words, the tissue collapse visibility distance T refers to a distance from the cutting head of water jet that has not been or is less affected by the obstruction caused by the compression and the deformation of the tissues.

The tissue collapse visibility distance T may be a numerical range, or may be a set of dynamically changing values related to the position of the water jet, that is, different tissue collapse visibility distances T can be determined for different fixed positions P of the water jet, and an array corresponding to the tissue collapse visibility distance T and the position P of the water jet can be stored.

Further, the following movement position trajectory parameters of the endoscope can be determined based on the tissue collapse visibility distance T, the linear start position Cz_start = z_start + T and the linear stop position Cz_stop = z_stop + T of the endoscope in the linear following movement position trajectory parameters of the endoscope are set. In this way, it can be ensured that in the following movement mode, the endoscopic field of view is not affected by the tissue collapse effect.

More preferably, the present application can determine the following movement trajectory parameters of the endoscope based on both the tissue collapse visibility distance T and the optimal endoscope operation distance L. For example, Cz_start = z_start + min(L, T) is set, and Cz_stop = z_stop + min(L, T) is set. In this way, it can be ensured that in the following movement mode, the endoscopic field of view is not affected by the tissue collapse effect, and a better imaging effect can be achieved.

Further, in the present application, the endoscopic depth-of-field delta can also be set and adjusted, and the distance between the endoscope lens and the cutting head of the water jet can be appropriately adjusted within an allowable range of the the endoscopic depth-of-field delta, thereby ensuring that the water jet and the water jet stream sprayed by the water jet fall into the endoscopic field of view as much as possible. The endoscopic depth-of-field delta is an operatable range of the endoscope before and after the aforementioned optimal endoscope operation distance L, that is, a depth of field of the endoscope when a focus is clearest.

With the above arrangement, when the water jet is actually operated, the endoscope can start the following movement mode to drive the endoscope to move along the following movement position trajectory. Since the following movement position trajectory parameters of the endoscope are determined based on the planned movement position trajectory parameters of the water jet, the linear movement of the endoscope moving according to the following movement position trajectory parameters and the linear movement of the water jet moving according to the planned movement position trajectory parameters can always be kept synchronous, thereby ensuring that the endoscope can still clearly observe the cutting head of the water jet, the water column sprayed therefrom, and surrounding tissue regions even after the water column spray and the surrounding tissue collapse effect are taken into account. In addition, since the following movement mode of the endoscope is preset, the following movement position trajectory parameters of the endoscope are also determined in advance based on the planned movement position trajectory parameters of the water jet, so that the endoscope is not required to perform a large amount of image data calculation such as tracking when performing the following movement, which greatly reduces the calculation amount of the control unit or the processor during the movement of the endoscope. Therefore, the control unit or the processor can have sufficient computing power to calculate and analyze the actual movement position trajectory parameters of the water jet during the operation thereof based on the endoscopic images, achieving a technical effect of near real-time monitoring. A specific monitoring method is described in detail below.

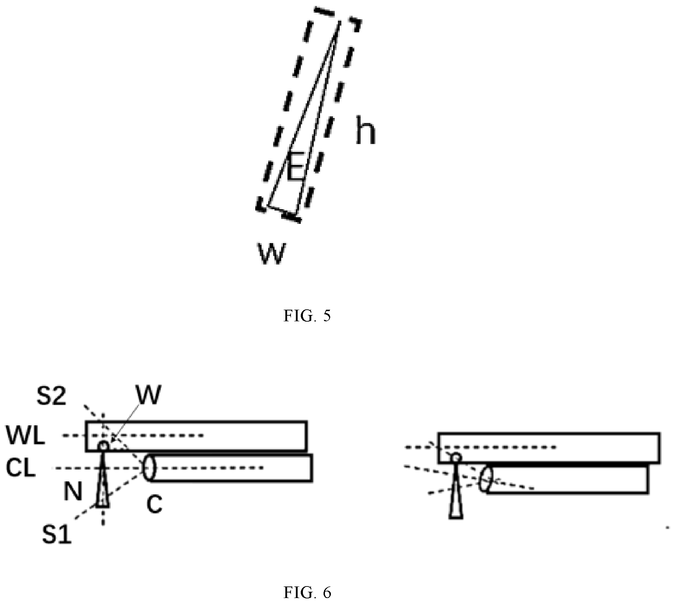

The endoscope in the following movement mode can acquire in real time the endoscopic images of the cutting head of the water jet and the sprayed water column, and as described above, by using the following movement trajectory determination method of the present application, the images of the cutting head of the water jet and the water column can be clearly identified from the acquired endoscopic images. As shown in FIG. 5, the control unit or the processor determines a water column region in each of the acquired endoscopic images by using an image segmentation algorithm, and calculates a length of the water column. The obtained length of the water column is the jet length trajectory parameter R in the actual movement position trajectory parameters of the water jet. Since the images are clear and the image segmentation algorithm is very mature, a calculation result of the jet length trajectory parameter R is obtained almost in real time.

For example, a water column region E and a bounding polygonal region can be obtained by separating the water column region E from the endoscopic image. By measuring the bounding polygonal region, for example, by measuring a height h and a width w of a minimum bounding rectangle in the image, an actual action range w and length h of the water jet stream can be determined and monitored. The length h is an actually measured jet length trajectory parameter, and by comparing the measured length h with the planned jet length trajectory parameter R, whether the actually measured jet length trajectory parameter of the water jet in the step is consistent with the planned jet length trajectory parameters thereof can be evaluated.

Further, the control unit or the processor may analyze the endoscopic images acquired in the following movement mode to calculate the rotational movement position trajectory parameters in the actual movement position trajectory parameters of the water jet. In the present application, by measuring and calculating a swing angle and/or a swing frequency of the water column, the rotational movement position trajectory parameters of the water jet are monitored. Further, different methods for monitoring the rotational movement position trajectory parameters of the water jet are used based on a comparison result between a rotation angle range and a tissue collapse visibility angle range in a preset trajectory of the water jet.

### Measuring and Calculating Swing Angle to Evaluate Actual Rotational Movement Position Trajectory Parameters of Water Jet

When the entire water column can be observed through the endoscope, rotation angle trajectory parameters of the water jet can be directly obtained by measuring and calculating the swing angle range of the water column, for example, a series of endoscopic image frames can be obtained, and the swing angle of the water column can be calculated by segmenting a water column image of each frame.

Specifically, in a movement process of a step, separated water column images of all frames from the start to the end of the movement in the step are recorded, angles between long axes of water columns in all water column images and a vertical direction are calculated with a vertically downward angle from the cutting head of the water jet as 0 degrees, so as to obtain a minimum swing angle RotateStartReal and a maximum swing angle RotatestopReal of each of the long axes of the water columns in all the water column images during the movement in this step. (RotateStartReal, RotatestopReal) represents the swing angle range, which is an actually measured rotation angle range of water column movement in the step. By comparing the actually measured rotation angle range (RotateStartReal, RotatestopReal) of the water column movement in the step with the planned angle range (θ_start, θ_stop) determined by the rotational movement trajectory parameters of the step, whether the actually measured rotational movement position trajectory parameters of the water jet in the step are consistent with the planned rotational movement position trajectory parameters can be evaluated.

However, the above is only an ideal case, and it is found in the actual observation process that although the tissue collapse visibility distance is considered when the following movement position trajectory parameters are determined, the field of view will still be obstructed to some extent by the compressed tissue during the actual observation, and in this case, the swing angle of the water jet may exceed the endoscopic field of view, that is, a full swing angle range of the water jet cannot be observed only from the endoscopic field of view. FIG. 2 is a schematic cross-sectional view of the water jet of the present application, W represents a center of the water jet, C represents a center of the endoscope, a solid-line circle represents a radiation range of the water jet stream, and a dashed-line circle represents the endoscopic field of view. Due to structural limitations of the water jet and the endoscope, the radiation range of the water jet stream and the endoscopic field of view are usually not 360 degrees, but sector regions less than 360 degrees. As shown in FIG. 2, the radiation range of the water jet stream is a sector region PWQ, and the endoscopic field of view is a sector region PCQ. As shown in FIG. 3, due to an obstruction of an own structure (such as an upper sheath) of the water jet and the impact of the collapse of the surrounding tissues, a part of the radiation range of the water jet stream may not fall within the endoscopic field of view, which causes incomplete imaging due to the obstruction when the water jet and the sprayed water jet stream are observed through the endoscope.

In this case, if the foregoing manner for calculating the swing angle of the water jet is still used, a problem of inaccurate monitoring results caused by a data loss inevitably exists.

In order to solve the above problems, the present application first defines a tissue collapse visibility angle, and selects different methods for monitoring rotational movement position trajectory parameters according to different ranges of tissue collapse visibility angle.

Specifically, the tissue collapse visibility angle is defined by Tθ_start and Tθ_stop. A method for determining Tθ_start and Tθ_stop is as follows: at the tissue collapse visibility distance T, start and stop angles of tangent lines between the water column E, which is centered on the cutting head of the water jet and has a planned jet length R as a radius, and the boundaries of surrounding collapsed tissues. The image segmentation algorithm can be used for determining regions A, B, C, D, E, and F in FIG. 3 from the real-time images of the endoscope, thereby determining the tissue collapse visibility angle (Tθ_start, Tθ_stop) at the tissue collapse visibility distance T.

The present application also defines a parameter waterjet_visible, which is used for indicating whether the water jet stream can be imaged by the endoscope. By comparing the planned angle range (θ_start, θ_stop) of the water jet with a tissue collapse visibility angle (Tθ_start, Tθ_stop) determined based on endoscopic image segmentation, an assignment of the parameter waterjet_visible is determined according to a comparison result.

For example, taking the vertically downward angle from the cutting head of the water jet as 0 degrees, when Tθ_start ≤ θ_start and Tθ_stop ≥ θ_stop, which means that the water column within the rotational movement range of the step can be observed through the endoscope, waterjet_visible = 2 is set; when Tθ_stop ≤ θ_start, or Tθ_start ≥ θ_stop, which means that any water column within the rotational movement range of the step cannot be observed through the endoscope, waterjet_visible = 0 is set; and when θ_start < Tθ_stop < θ_stop, or θ_start < Tθ_start < θ_stop, which means that a part of the water column within the rotational movement range of the step can be observed through the endoscope, waterjet_visible = 1 is set.

### Measuring and Calculating Swing Frequency of Water Column to Evaluate Actual Rotational Movement Position Trajectory Parameters of Water Jet

When waterjet_visible = 1, the swing frequency of the water column can be measured and calculated to evaluate the actual rotational movement position trajectory parameters of the water jet, that is, to evaluate whether the water jet performs an operation along the planned movement trajectory.

An observed angular position CameraObserve_degree is defined, which is an angular position of a swing position of the water column observed in a selected endoscopic image, and the angular position is used for observing and calculating an actual swing frequency of the water column. CameraObserve_degree is a directional vector that includes both an angle value of the water column swinging to that position and a direction or trend of a change in the angle value of the water column swinging to that position.

The water jet performs resection in each step in a sector-sweeping pattern, and after entering the next step, performs the resection in the sector-sweeping pattern in an opposite direction. Therefore, when the water jet performs resection in a plurality of successive steps, the water column moves in a spiral-like pattern, and the rotation (swing) in each step has a period (frequency) related to the aforementioned V_{θ}, θ_start, and θ_stop. The swing frequency of the water column referred to herein is calculated as a reciprocal of a sum of movement times of the two among the steps. When movement changes between adjacent steps are continuous rather than sudden, a frequency of the water jet swinging through the observed angular position can be approximated as half the reciprocal of the movement time (required to move from θ_start to θ_stop) of one of the steps. An image acquisition frequency of the endoscope is much greater than the swing frequency of the water column. Therefore, by acquiring the observed angular positions and calculating swing frequencies thereof, whether the water jet performs the operation along the planned rotational movement trajectory can be determined.

First, the rotational movement trajectory parameters of the water jet in the planned movement trajectory of a current step are acquired, the parameters including the rotational start angle θ_start of the water jet in the step, the rotational stop angle θ_stop of the water jet in the step, and the rotational speed V_{θ} of the water jet in the step. Based on the planned rotational movement trajectory parameters, a planned swing frequency θ_f of the water column in the step is calculated, that is, θ_f = 0.5 * V_{θ}/(θ_stop - θ_start).

Separated water column images of all frames from the start to the end of the movement in the step are recorded, an interval between arrival times of the water column at adjacent observed angular positions (CameraObserve_degree) is acquired by separating the water column image of each frame, that is, a time interval between the two adjacent CameraObserve_degree positions having the same water column angle value and the same change direction thereof is determined, and a reciprocal of the time interval is an actual swing frequency θ_fReal of the water jet.

Due to periodic movement of the water jet, two observed angular positions (CameraObserve_degree) having the same water column angle value and the same change direction thereof are usually separated by an odd number of steps. Theoretically, two CameraObserve_degrees having the same angle value and the same change direction thereof which are separated by an odd number of steps can be used for calculating the swing frequency. However, considering continuity of a resection channel, it is preferable to calculate the swing frequency by two observed angular positions (CameraObserve_degree) having the same angle value and the same change direction thereof which are separated by one step. Here, the two observed angular positions (CameraObserve_degree) having the same angle value and the same change direction thereof which are separated by one step are referred to as the adjacent observed angular positions, the time interval between the adjacent observed angular positions is calculated, and a reciprocal of the time interval is the actual swing frequency θ_fReal of the water jet in the step.

By comparing the actual swing frequency θ_fReal of the water column movement in the step with the planned swing frequency θ_f determined based on the rotational movement trajectory parameters of the step, a deviation between the two can be determined, and whether the actually measured rotational movement position trajectory parameters of the water jet in the step are consistent with the planned rotational movement position trajectory parameters is evaluated.

### Measuring and Calculating Swing Frequency of Key Point of Lance to Evaluate Actual Rotational Movement Position Trajectory Parameters of Water Jet

For extreme cases, when waterjet_visible = 0, that is, when any water column within the rotational movement range of the step cannot be observed through the endoscope, a value determined by a method for measuring and calculating a swing frequency of a key point of the lance can be used as a water column swing frequency to evaluate the actual rotational movement position trajectory parameters of the water jet.

Specifically, as shown in FIG. 3, the region A is a water jet region, a key point is identified and marked for a separated image of the region A by using a key point detection algorithm such as an SIFT key point. By sampling the endoscopic images, an interval between arrival times of the identified key point at the adjacent observed angular positions (CameraObserve_degree) having the same observed angle value and the same change direction thereof is determined, and the reciprocal of the time interval is used as the actual swing frequency θ_fReal of the water column. Further, by comparing the actually measured swing frequency θ_fReal of the water column movement in the step with the planned swing frequency θ_f determined based on the rotational movement trajectory parameters of the step, a deviation between the actual swing frequency of the water column and the planned swing frequency thereof can be determined, and whether the actually measured rotational movement position trajectory parameters of the water jet in the step are consistent with the planned rotational movement position trajectory parameters thereof is evaluated.

Among the above methods for measuring, calculating, and evaluating the actual rotational movement trajectory parameters of the water jet, the method for measuring and calculating the swing frequency of the key point of the lance can also be used in a case of waterjet_visible = 1 or 2, and the method for measuring and calculating the swing frequency of the water column can also be used in the case of waterjet_visible = 2. However, in order to achieve higher calculation speed and accuracy, based on a comparison result between the rotation angle range of the preset trajectory of the water jet and the tissue collapse visibility angle range, different methods for monitoring the rotational movement position trajectory parameters of the water jet are selected for different values assigned to waterjet_visible, that is, when waterjet_visible = 2, the method for measuring and calculating the swing angle is selected, when waterjet_visible = 1, the method for measuring and calculating the swing frequency of the water column is selected, and when waterjet_visible = 0, the method for measuring and calculating the swing frequency of the key point of the lance is selected.

### Rotational Movement of Endoscope

In most cases, the endoscope lens is parallel to an axial direction of the water jet (that is, faces directly forward). However, when the optimal endoscope operation distance L is determined, the water column E used for a test is usually directed downward or only within a certain angle range, but a rotation range of the water column during an actual operation may exceed the endoscopic field of view.

For example, as shown in a left figure in FIG. 6, when the endoscope lens is parallel to the axial direction of the water jet (that is, faces directly forward), a visible angle of the endoscope is an angle S2-C-S1, but the rotation range of the water column exceeds the endoscopic field of view during the actual operation, so that the water column cannot be observed through the endoscope lens, that is, waterjet_visible = 0. When the water column cannot be observed through the endoscope lens, although the aforementioned alternative calculation methods may be used, accuracy thereof may be low. Therefore, an alternative solution may be used, that is, the rotational movement of the endoscope is increased, a rotatable endoscope lens is used, as shown in a right figure in FIG. 6, an endoscope lens having a certain tilt angle is used, and the tilt angle of the endoscope lens is adjusted as the endoscope rotates, so that the rotated endoscope can meet a visible angle requirement of the endoscope within a limited distance determined based on min(L, T).

For the case in which the tilt angle of the endoscope lens is adjustable, the following movement of the endoscope may be set to include a rotational movement, that is, the following movement mode of the endoscope is set to not only follow the linear movement of the water jet, but also follow the rotational movement thereof, and correspondingly, the following movement position trajectory parameters further include Cθ_start representing a rotational start angle, Cθ_stop representing a rotational stop angle, and CV_{θ} representing a rotational speed of the endoscope in the step. A purpose of the following movement of the endoscope is to adjust an endoscopic field-of-view region to ensure that the entire water jet and sprayed water column are observed as much as possible. At this time, it is also necessary to determine Cθ_start, Cθ_stop, and CV_{θ} of the endoscope based on θ_start, θ_stop, and V_{θ} of the water jet.

Through the above steps, the actual rotational movement position trajectory parameters and jet length trajectory parameters during the operation of the water jet can be measured and calculated based on the endoscopic images, and whether there are deviations from the planned rotational movement position trajectory parameters and jet length trajectory parameters is determined, and when there are deviations, an alarm mechanism can be activated or the deviations can be fed back to the control unit, and a planned trajectory of the water jet can be compensated and adjusted based on a feedback result.

### Monitoring Safety of Sensitive Part

Referring to FIG. 4, FIG. 4 schematically illustrates a sensitive part in a lumen of a patient. In the figure, C represents an approximate center of the endoscope, A represents an image of the water jet in the endoscopic field of view, B, D, and F represent the tissues compressed and deformed by the water jet or other tissues within the lumen, and E represents the water jet stream. In a prostate surgery scenario, F is a tissue of the sensitive part in the lumen, such as a ureteral orifice or verumontanum, and the tissue of the sensitive part should not be resected during surgery to avoid damage to the patient.

Generally, when the planned movement trajectory of the water jet is determined based on preoperative images or the ultrasound images, avoidance of resection of the sensitive part has been considered. However, due to accuracy of the ultrasound images or movement of an organism, an actual position of the sensitive part of the organism may deviate from a recognition result of the preoperative images or the ultrasound images during actual execution of the surgery, resulting in a risk of being injured based on the planned scheme.

In the present application, the endoscope in the following movement mode can acquire clear real-time images of the water jet and the water column, and based on the acquired real-time images, a safety protection function of the sensitive part is added. An implementation idea thereof includes monitoring safety of the sensitive part based the jet length trajectory parameters, or monitoring safety of the sensitive part based on the rotational movement position trajectory parameters.

### Monitoring Safety of Sensitive Part based on Jet Length Trajectory Parameters

First, the clear real-time images are obtained through the endoscope in the following movement mode, a sensitive part F in the endoscope image is identified by using the image segmentation algorithm, and a shortest distance S1 between a contour boundary of the identified sensitive part F and the cutting head of the water jet is measured and calculated.

Further, a safe resection depth for the sensitive part F is determined based on the shortest distance between the contour boundary of the sensitive part F and the cutting head of the water jet. Preferably, the safe resection depth is further determined based on a preset safe removal distance. The safe removal distance is determined in advance through experiments or preset based on factors such as experience or surgical requirements. Setting of the safe removal distance contributes to providing a larger margin to ensure safety without the water jet stream touching the sensitive part F. For example, if the safe removal distance is set to S2, the resection depth of the water jet is required to stop at a position that is S2 away from a contour of the sensitive part F. Further, when the shortest distance between the contour boundary of the sensitive part F and the cutting head of the water jet is S1, and the safe removal distance is set to S2, the safe resection depth of the sensitive part F is determined as S1 - S2.

Further, the planned movement trajectory of the water jet is traversed, for example, the calculated planned jet length trajectory parameter in a certain step is greater than the aforementioned safe resection depth of the water jet, indicating that the water jet may subsequently resect the sensitive part F. In this case, a surgery execution instrument 100 issues an alarm to suspend the resection. At this time, planning of the water jet can be readjusted to avoid the sensitive part or adjust the safe removal distance S2 for the sensitive part, so as to avoid safety problems, surgical failures, and the like caused by resecting the sensitive part. For example, based on a monitoring result, a distance by which the jet length is required to be reduced in a planned step of the water jet at this position which corresponds to the endoscopic step is obtained, and the planned trajectory of the water jet is dynamically adjusted by adjusting the jet length trajectory parameter R in the actual movement position trajectory parameters in the planned step of the water jet.

### Monitoring Safety of Sensitive Part based on Rotational Movement Position Trajectory Parameters

According to another embodiment, a non-removal region can be set for the sensitive part. Taking FIG. 4 as an example, F is the sensitive part, E to E' is the planned removal region, and D is a removal target. The non-removal region C is defined as a sector region centered on the water jet A and including the sensitive part F, and may be expressed by (Sθ_start, Sθ_stop), in which Sθ_start represents a start angle of a tangent line of the contour of the sensitive part with the water jet A as the center, and Sθ_stop represents a stop angle of the tangent line of the contour of the sensitive part with the water jet A as the center. In this case, when the water jet is directed toward the sensitive part F, no water jet stream is sprayed, which means that a tissue in the non-removal region C (Sθ_start, Sθ_stop) is not ablated or resected at all. With respect to this solution, the safety of the sensitive part can be monitored based on the rotational movement position trajectory parameters. Specific implementation steps are as follows:
First, the clear real-time images are obtained through the endoscope in the following movement mode, the sensitive part F in the endoscopic image is identified by using the image segmentation algorithm, and an actual non-removal region (Sθ_start, Sθ_stop) is calculated.

Further, whether the non-removal region C overlaps with the planned removal region EE', that is, whether (Sθ_start, Sθ_stop) overlaps with (θ_start, θ_stop) is determined, and safety strategies such as issuing an alarm, suspending resection, or adjusting a plan, are executed based on the presence or degree of the overlap.

Preferably, a safe overlap range U can also be set. The safe overlap range U may be an area size, that is, when an overlap area between the non-removal region C and the planned removal region EE' is greater than a certain threshold, it is considered that there is a risk. In addition, the safe overlap range U may also be a magnitude of an overlap ratio. For example, when a ratio of the overlap area of the non-removal region C and the planned removal region EE' to the non-removal region C is greater than a certain threshold, it is considered that there is a risk.

When it is determined that there is a risk, the planning of the water jet can be readjusted to avoid the sensitive part F or to adjust the safe overlap range U of the sensitive part F, so as to avoid safety problems, surgical failures, and the like caused by resecting the sensitive part F. Simultaneously, based on the non-removal region C obtained in real time from the endoscopic images, a relationship C ∩ EE' in planning of the water jet in each of subsequent steps can be calculated in advance as real-time feedback on a resection range of the planned movement trajectory of the water jet, and the resection range of the planned movement trajectory of the water jet in subsequent steps is automatically adjusted to achieve dynamic adjustment of a planned resection trajectory of the water jet.

In the foregoing solutions, the alarm mechanism may be triggered based on monitoring of the actual movement position trajectory parameters of the water jet and/or the actual sensitive part. In particular, the alarm mechanism in the present application is set based on real-time monitoring of the endoscope in the following movement mode, and in an actual surgical scenario, the linear movement trajectory of the cutting head of the water jet is from front to back, and a tissue region (including the resection range, the sensitive part, and the like) observed through the endoscope located behind the cutting head of the water jet includes a region to be resected by the cutting head of the water jet, thereby enabling prediction of the deviation and the risk, achieving an early warning function, and contributing to providing better planning solutions and safety guarantee.

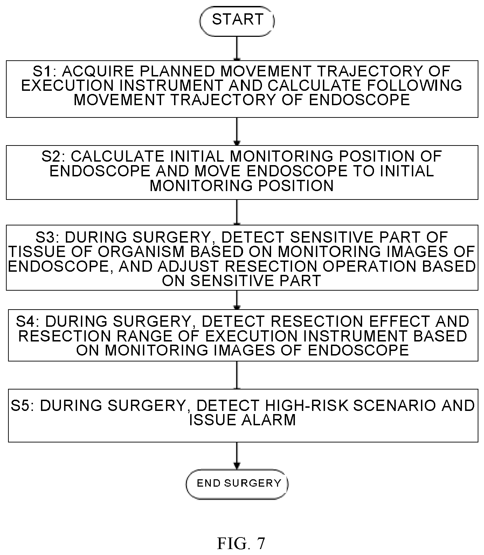

Next, one of flows of performing the resection surgery by the surgery execution instrument of the present application is described with reference to FIG. 7. Those skilled in the art can understand that the flow described below is only an application of the present application, and the order and specific content of the steps may be appropriately set according to actual scenarios.

In step S1, the planned movement trajectory of the water jet is acquired, and the following movement trajectory of the endoscope is calculated.

In step S2, an initial monitoring position of the endoscope is calculated, and the endoscope is moved to the initial monitoring position.

In step S3, during the surgery, the sensitive part of the tissue of the organism is detected based on monitoring images of the endoscope, and a subsequent resection plan is adjusted based on the sensitive part.

In step S4, during the surgery, the actual movement position trajectory parameters of the water jet are monitored based on the monitoring images of the endoscope, and the subsequent resection plan is adjusted based on the deviation between the actual movement position trajectory parameters and the planned movement position trajectory parameters.

In step S5, during the surgery, a high-risk scenario is monitored and an alarm is issued. Here, the high-risk scenario refers to cases in which, for example, the time during which both the water jet and the water column are simultaneously lost within the endoscopic field of view exceeds a specified threshold, or the swing frequency or the resection depth of the water jet exceeds a safety threshold relative to the planned frequency or resection depth.

Through the above embodiments of the present application, the technical problems in the background art can be effectively solved, and effective, accurate and real-time monitoring is performed on an execution situation during the surgical procedure using the water jet. The present application is particularly applicable to an automatic water jet system, and when the water jet moves and is operated according to the planned movement position trajectory parameters, the solutions of the present application can monitor in real time the actual movement position trajectory parameters of the water jet, which is beneficial for real-time adjustment of the movement position trajectory parameters. The present application can also monitor in real time an actual operation scenario of the water jet, especially a region involving the sensitive part, determine a safety risk of the planned movement trajectory based on a real-time monitoring result, and trigger the alarm mechanism in time to take emergency measures.

## Claims

1. An endoscope-based monitoring apparatus for a water jet movement trajectory, the apparatus comprising:
a water jet, an endoscope, a water jet driving unit, an endoscope driving unit, and a control unit, wherein
the water jet driving unit and the endoscope driving unit are both electrically connected to the control unit,
when driven by the water jet driving unit, the water jet is capable of moving along a planned movement trajectory and spraying a water jet stream to perform tissue resection or ablation, the water jet movement trajectory being defined by water jet movement position trajectory parameters, which include linear movement position trajectory parameters, and
when driven by the endoscope driving unit, the endoscope moves and has a following movement mode in which the endoscope is capable of following the water jet along a following movement trajectory to perform at least linear movement and monitoring actual movement position trajectory parameters of the water jet, the following movement trajectory being defined by following movement position trajectory parameters, which at least include linear following movement position trajectory parameters and are determined by the control unit based on planned movement position trajectory parameters of the water jet.

2. The monitoring apparatus for a water jet movement trajectory according to claim 1, wherein
the water jet movement position trajectory parameters further include rotational movement position trajectory parameters and/or jet length trajectory parameters.

3. The monitoring apparatus for a water jet movement trajectory according to claim 1, wherein
linear movement directions of the endoscope and the water jet are parallel to each other, and the endoscope is located at a rear portion of the water jet, with a predetermined distance S maintained between an observation lens of the endoscope and a water jet spray portion.

4. The monitoring apparatus for a water jet movement trajectory according to claim 1, wherein
the water jet movement trajectory is defined as a combination of arrays containing the water jet movement position trajectory parameters for a plurality of steps, the following movement position trajectory of the endoscope is also defined as a combination of arrays containing the following movement position trajectory parameters for the same number of steps, and the control unit determines, based on the water jet movement position trajectory parameters included in each array for one step, the following movement position trajectory parameters of the endoscope in a step corresponding to the step.

5. The monitoring apparatus for a water jet movement trajectory according to claim 2, wherein
the endoscope acquires in real time endoscopic images of a cutting head of the water jet and a water jet stream in the following movement mode, and the control unit analyzes the acquired endoscopic images and determines the jet length trajectory parameters and/or the rotational movement position trajectory parameters in the actual movement position trajectory parameters of the water jet.

6. The monitoring apparatus for a water jet movement trajectory according to claim 2, wherein
the control unit determines the following movement position trajectory parameters based on a tissue collapse visibility distance and/or an optimal endoscope operation distance.

7. The monitoring apparatus for a water jet movement trajectory according to claim 2, wherein
the control unit performs image segmentation processing on an endoscopic image of water jet movement acquired in real time by the endoscope, and monitors the jet length trajectory parameters.

8. The monitoring apparatus for a water jet movement trajectory according to claim 2, wherein
the control unit monitors, by measuring and calculating a swing angle and/or a swing frequency of a water column, the rotational movement position trajectory parameters of the water jet.

9. The monitoring apparatus for a water jet movement trajectory according to claim 8, wherein
the control unit uses, based on a comparison result between a rotation angle range in a preset trajectory of the water jet and a tissue collapse visibility angle range, different monitoring methods for the rotational movement position trajectory parameters of the water jet.

10. The monitoring apparatus for a water jet movement trajectory according to claim 9, wherein
when the entire water column is observable through the endoscope, the control unit monitors and evaluates, by measuring and calculating a swing angle range of the water column in an endoscopic image, whether actual rotational movement position trajectory parameters of the water jet are consistent with planned rotational movement position trajectory parameters.

11. The monitoring apparatus for a water jet movement trajectory according to claim 9, wherein
when the water jet stream is within an endoscopic field of view, the control unit calculates a swing frequency of the water jet based on water jet stream images from the same endoscopic observation angle at an even-numbered step interval among monitoring images of the endoscope, and when the water jet stream is not within the endoscopic field of view, the control unit calculates the swing frequency of the water jet based on feature point images of the water jet from the same endoscopic observation angle at an even-numbered step interval among observation images of the endoscope.

12. The monitoring apparatus for a water jet movement trajectory according to claim 11, wherein
the control unit evaluates, by comparing an actual swing frequency of water column movement in a step with a swing frequency determined by rotational movement trajectory parameters in the step, whether actual rotational movement position trajectory parameters of the water jet in the step are consistent with planned rotational movement position trajectory parameters.

13. The monitoring apparatus for a water jet movement trajectory according to claim 1, wherein
the endoscope includes a lens having an adjustable tilt angle, and following movement trajectory parameters of the endoscope include rotational following movement position trajectory parameters.

14. The monitoring apparatus for a water jet movement trajectory according to claim 1, wherein
the endoscope further performs safety monitoring on a sensitive part in the following movement mode.

15. The monitoring apparatus for a water jet movement trajectory according to claim 14, wherein
the control unit determines a safe resection depth based on an image acquired by the endoscope, and determines a risk based on a comparison result between a planned jet length trajectory parameter and the safe resection depth.

16. The monitoring apparatus for a water jet movement trajectory according to claim 15, wherein
the control unit determines, based on the image acquired by the endoscope in the following movement mode, the sensitive part, and determines the safe resection depth for the sensitive part based on a shortest distance between a contour boundary of the sensitive part and a cutting head of the water jet.

17. The monitoring apparatus for a water jet movement trajectory according to claim 16, wherein
the control unit further determines the safe resection depth based on a preset safe removal distance.

18. The monitoring apparatus for a water jet movement trajectory according to claim 14, wherein
the control unit performs the safety monitoring on the sensitive part based on rotational movement position trajectory parameters, determines a non-removal region based on an image acquired by the endoscope, and determines a risk based on a comparison result between planned rotational movement position trajectory parameters and the non-removal region.

19. The monitoring apparatus for a water jet movement trajectory according to claim 1, wherein
the endoscope further has an autonomous movement mode in which movement of the endoscope and movement of the water jet are independent of each other, and in the autonomous movement mode, the endoscope is driven to move and parameters are acquired, and the control unit sets or updates the following movement position trajectory parameters based on the acquired parameters.

20. The monitoring apparatus for a water jet movement trajectory according to claim 1, wherein
the endoscope further includes an image acquisition unit configured to acquire an image and transmit the image to the control unit, and the control unit analyzes acquired image data and adjusts the planned movement trajectory of the water jet and the following movement trajectory of the endoscope based on an analysis result.
